# EUROPEAN PATENT APPLICATION

(11) **EP 2 241 199 A2**
(43) Date of publication of application: **20.10.2010**
(21) Application number: 09709828.9
(22) Date of filing: 12.02.2009
(51) Int. Cl.: A23L 1/30

(54) **METHOD FOR PRODUCING FERMENTED PRODUCT USING NATURAL MATERIAL, AND FOOD OR MEDICINE CONTAINING FERMENTED PRODUCT MADE FROM SAME**

(30) Priority: 14.02.2008 KR 20080013299
(71) Applicant: Barley & Oats Co., Ltd, Jeollanam-do 513-832 (KR)
(72) Inventor: CHO, Seog Ho, Jeonju-si Jeollabuk-do 560-170 (KR); KIM, Chul Jin, Seongnam-si Gyeonggi-do 463-420 (KR); PARK, Jee Won, Jeongeup-si Jeollabuk-do 580-812 (KR); KIM, Tae Hui, Gunsan-si Jeollabuk-do 573-775 (KR); JANG, Jeong Hoon, Anyang-si Gyeonggi-do 431-070 (KR)
(74) Representative: Albrecht, Thomas
(86) International application number: PCT/KR2009/000656
(87) International publication number: WO 2009/102152

(57) **Abstract**

The present invention relates to a method for producing a fermented product of natural materials comprising nucleic acids in an amount of 3 to 4g or less on the basis of 70g of protein content or 24g of dietary fiber content in the fermented product by adding microorganisms and fermenting the mixture to reduce carbohydrate content and to increase beta-glucan content in natural materials, a fermented product of natural materials produced by said method, and a food product or a medicine product which comprises said fermented product.

## Description

### Technical Field

The present invention relates to a method for producing a fermented product of natural materials comprising nucleic acids in an amount of 3 to 4g or less on the basis of 70g of protein content or 24g of dietary fiber content in the fermented product by adding microorganisms for fermentation to reduce carbohydrate content and to increase beta-glucan content in natural materials, a fermented product of natural materials produced by said method, and a food product or a medicine product which comprises said fermented product.

### Background Art

Unlike the past, many people in the modern world suffer from obesity due to excessive calorie intake and lack of exercise, or they are exposed to very high risk of being obese. It is clear that obesity, which is so called a modern-day disease, is mainly caused by intake of excessive calories and shift of a human work from physical labor to mental labor.

Once exposed to a symptom of obesity, complications such as a heart problem, hypertension, diabetes and the like can occur and it also becomes a mental burden to people in the modern world who wish to maintain their physical beauty in addition to having basic necessities including clothing, food and housing. According to statistics, with obesity spreading far and wide, it is found that in Korea 19.9% adult male and 23.7% adult female suffer from metabolic syndrome in 1998. For Americans and Europeans who usually have in their diet more animal proteins enriched with fats, it is expected that the ratio is much higher.

In order to prevent obesity, it is needless to say that intake of an appropriate amount of calories and proper exercise are required. However, for a human, eating desire is very hard to control and also exercising appropriately during busy days of the modern life is a very difficult thing to achieve. Further, although healthy diet should comprise a wide variety of nutritional components, in real life it is also very difficult to have each meal prepared based on precise calculation of nutritional values. When people have an excess amount of fats and carbohydrates as they become old, an excess body fat will accumulate on their abdomen and hip regions, directly leading to obesity. In particular, when fats are accumulated on an abdomen region, a severe metabolic syndrome might be caused.

According to the results of a scientific research, the amount of carbohydrates which can be stored in a human body is limitative, i.e., around 1,000 to 2,000Kcal. However, stored fats may count several tens of thousand calories. Thus, excess carbohydrates are easily converted into neutral fat form, and mainly accumulated around intestines. On the other hand, even when being taken in a rather large amount, proteins are accumulated in a human body in a relatively small amount compared to carbohydrates and fats. Still, when taken in an amount greatly in excess of the normally required, proteins are also accumulated in a human body, and as a result liver and kidney fatigue can be caused as a result of their metabolization.

Recently, importance of beta-glucan, which is a member of dietary fiber family, is suggested in relation with obesity and metabolic syndrome. This is because, not only it can reduce fat weight and the size of fat cells that are accumulated around intestines, but also it is effective for reducing content of neutral triglycerides comprised in a human body, that is related to obesity (Soon Ah Kang et al., 2002, J. Korean Soc. Food Sci. Nutr. 31(6), 1052-1057; Ho-Moon Seog et al., 2002, Korean J. Food Sci. Technol. Vol 34, No 4, PP 678-683; Jue Li, Takashi Kaneko et al., Metabolism, Vol 52, No 9 (September), 2003, pp1206-1210).

Furthermore, importance of protein intake has been suggested. During growth period, it is essential for a human to have a large amount of proteins. For middle aged people, appropriate protein intake is still important considering that body accumulation is relatively minor even when a little excessive amount of proteins is taken, long-last feeling may be obtained as they require a rather long digestion time in human body, and muscle mass dramatically decreases as people are getting older.

As mentioned in the above, preventing and treating human obesity appears to be quite simple. However, due to improper eating habit and lack of human will, population of obese people keeps growing and social and economic loss due to obesity appears to be much more significant than expected.

### Detailed Description of the Invention

### Technical Problems to be Solved

The present invention is devised to solve the problems described above. Specifically, according to the present invention, a food product or a medicine product having relatively low calories, low content of carbohydrates and fats, and high content of beta-glucan is provided, wherein the product is produced by a process of controlling nucleic acids not to incur any problem when it is taken in a large amount while at the same time the content of carbohydrates, which can easily accumulate in a human body, is reduced, flavor and taste are improved and content of beta-glucan is increased by fermentation using microorganisms, and as a result disadvantages of natural materials, which can be effectively taken by people who suffer from obesity or metabolic syndrome or who wish to prevent them, can be overcome.

### Means for Solving the Technical Problems

In order to solve the problems described above, according to the present invention, a method for producing a fermented product of natural materials, in which microorganisms are added to natural materials for fermentation to increase content of beta-glucan while decreasing carbohydrate content and to control content of nucleic acids, is provided.

Further, according to the present invention, a fermented product which is prepared by said method is provided.

Still further, according to the present invention, a food product or a medicine product comprising said fermented product is provided.

### Advantageous Effect of the Invention

According to the present invention, it is possible to convert various kinds of natural materials, which have been regarded as a low-grade foodstuff as having less appealing flavor and taste compared to other food products, into a high-end product which comprises high content of beta-glucan and favorable flavor and taste and can provide full-stomach feeling even when it is taken in only a small amount.

Various types of functional foods relating to obesity, that are presently on the market, are rather expensive compared to effective ingredients actually comprised therein and they are produced mainly by an artificial process. However, since the product of the present invention is produced by a fermentation process, i.e., a natural process, it is quite cheap in terms of effective components comprises therein. Further, as having excellent stability, it can be safely and conveniently consumed by all people who suffer from obesity or metabolic syndrome, or wish to prevent them.

In particular, according to the present invention, for a patient who is suffering from serious obesity or metabolic syndrome, and thus requires an urgent treatment, an extremely low calorie product that is mainly based on dietary fiber including beta-glucan can be prepared. For a patient suffering from less serious obesity or metabolic syndrome, or for people who wish to prevent them, a product which comprises both dietary fiber including beta-glucan and proteins at an appropriate level can be produced, and it can be generally consumed as a functional substitute for regular meals.

In future, the product of the present invention, which can be favorably and also conveniently consumed, may also serve as a stable demand for farmers who grow grain crops.

Considering that in future physical labors will be replaced by machines and humans will be mostly responsible for mental works in accordance with accelerated transformation of a society into the one mainly based on an intellectual services, a food product or a medicine product which is clearly different from those currently on the market will be required. In such case, the product of the present invention, which has an excellent flavor and taste and an easy digestion property, is also useful for prevention and treatment of a physical disorder such as obesity and metabolic syndrome caused by excessive mental works and can be consumed as a substitute for regular meals when a protein level is maintained at an appropriate level, and thus it will be an important food product and/or medicine product in future.

### Mode for Carrying out the Invention

In order to achieve the purpose of the invention described above, the present invention provides a method for producing a fermented product of natural materials comprising decreased carbohydrate content and increased beta-glucan content, including steps of:
- adding edible microorganisms to natural materials for fermentation; and
- controlling nucleic acids during or after the fermentation step.

More specifically, the present invention provides a method for producing a fermented product of natural materials comprising decreased carbohydrate content and increased beta-glucan content, including steps of:
- pulverizing and liquefying natural materials;
- adding edible microorganisms to the pulverized and liquefied natural materials obtained above for fermentation;
- controlling nucleic acids during or after the fermentation step;
- concentrating or drying the resulting fermented product in which nucleic acids are controlled; and
- adding texture, flavor and taste to the resulting concentrated or dried fermented product.

The method for producing a fermented product of natural materials according to the present invention may further comprise a step of saccharification after pulverizing and liquefying natural materials.

The method for producing a fermented product of natural materials according to the present invention comprises a step of adding edible microorganisms to the natural materials for fermentation. Examples of the edible microorganisms include fungus, yeast, bacteria and the like. Examples of fungus include *Fusarium* spp., *Aspergillus* spp., *Rhizopus* spp., and the like. Examples of yeast include *Saccharomyces* spp., and the like. Examples of bacteria include *Bacillus* and the like, but not limited thereto. Specific examples of the edible microorganisms described above can be *Fusarium venenatum, Aspergillus oryzae, Aspergillus awamori, Rhizopus microsporus, Saccharomyces cerevisiae, Bacillus megaterium, Bacillus subtilis* and the like, but not limited thereto.

Preferably, the microorganism is yeast. More preferably, it is *Saccharomyces cerevisiae.*

Fermentation method using the above-described microorganisms may vary depending on the types of microorganism used, fermentation condition and fermentation medium and the like. Fermentation condition and fermentation medium that are well known in the pertinent art can be used for the present invention. For example, yeast can be cultured at 30°C in YPD medium, and if necessary, an aerobic condition can be selected.

The above-described natural materials can be barley, wheat, triticale, rye, sorghum, oat, rice, corn, adlay, millet, hog millet, potato, sweet potato and the like, but not limited thereto. In addition, said natural materials can be mixed with each other or with other natural materials, and then used.

Said natural materials may include both water-soluble components and water-insoluble components. The water-insoluble components may impair flavor and taste of a food product. However, when they are fermented, if necessary, flavor and taste are improved and a digestion property of the food product can be also improved. Favorable components can be also generated during a fermentation process.

According to a method of one embodiment of the present invention, before fermentation, natural materials can be separated into water-soluble components and water-insoluble components, and then only water-soluble components can be subjected to a fermentation process. Water-insoluble components can be combined with a fermented product that is obtained after the fermentation process. When a fermentation process is carried out by using water-soluble components only, stirring and air flow inside a fermentation tank are improved so that fermentation efficiency can be increased and also separation of other useful substances along with the microorganisms at the time of controlling content of nucleic acids by removing the microorganisms from the fermented product after the termination of the fermentation process can be prevented.

Further, the method of the present invention comprises a step of controlling nucleic acids during or after a fermentation step. A method of controlling nucleic acids include a method of completing fermentation by minimizing a production amount of microorganisms during a fermentation step and a method of reducing content thereof by partially or completely removing the microorganisms from the fermented product that is obtained after the termination of the fermentation step.

As one way of minimizing the production amount of microorganisms during a fermentation process, there is a method in which fermentation is carried out while maintaining carbon/nitrogen ratio (i.e., C/N ratio) at relatively high level. Specifically, the C/N ratio mentioned above is in the range of 12.1 to 31.9. In such case, fermentation hardly occurs at initial stage and it takes a very long time for complete fermentation. For example, it may take up to 96 hrs to complete fermentation compared to normal time period of 48 hrs.

Still further, if necessary, based on a direct treatment of a fermented product obtained after the termination of a fermentation process, nucleic acids can be removed. Examples of a method of removing nucleic acids include laser irradiation, a heat shock treatment, sonication, alkaline lysis and the like, but not limited thereto.

In order to solve the problems associated with obesity and metabolic syndrome, the ultimate goal of the present invention is to develop a product which satisfies the following aspects.
1. It should give full-stomach feeling even when only a small amount is taken.
2. It should comprise beta-glucan in high content ratio so that it can be useful for prevention and treatment of metabolic syndrome.
3. It should comprise proteins at an appropriate level to be consumed as a substitute for regular meals.
4. It should have excellent taste and flavor to be repeatedly enjoyed by everybody.
5. It should have relatively low calories.

With respect to the first aspect, natural materials which can give full-stomach feeling even when only a small amount is taken have lots of diverse dietary fiber and proteins at medium level, and very small amount of fats, so that they are very appropriate to be used in the present invention in view of object of the invention described above. However, content of beta-glucan varies significantly among different natural materials.

Meanwhile, natural materials are found to be a food product which can be safely taken by people suffering from obesity or metabolic syndrome, because their calories per unit gram is relatively low and also nucleic acids content is very low.

However, it is also true that people tend to avoid such natural materials because they taste poor and content of carbohydrates, which can easily accumulate in human body, is high while content of beta-glucan is not high enough. Beta-glucan and other components comprised in such natural materials are compared to those comprised in other grain crops and the results are summarized in the following table. It was also confirmed that, depending on polishing level, variety and moisture content, content of each component may vary a little.

**Table 1**

| Comparison of major components comprised in several natural materials | | | | |
|---|---|---|---|---|
| | Barley | Oat | Millet | Sorghum |
| Carbohydrate | 64.0 | 57.0 | 68.0 | 66.1 |
| Fat | 2.1 | 4.7 | 3.9 | 2.8 |
| Protein | 12.0 | 14.3 | 9.7 | 9.6 |
| Fiber | 11.2 | 10.7 | 8.0 | 10.3 |

**Table 2**

| Comparison of composition ratio of beta-glucan comprised in several natural materials | | | | | | |
|---|---|---|---|---|---|---|
| Grain type | Anderson, et. al. (1978) | Henry (1985) | Joergensen, et. al. (1988) | McCleary & Glennie-Holmes (1985) | Prentice, et. al. (1980) | Lee, et. al. (2001) |
| Barley | 5.01 | 4.36 | 4.09 | 4.43 | 6.3 | 4.45 |
| Oat | 2.50 | 3.37 | 3.27 | 4.08 | 5.7 | 3.54 |
| Rice | 0.13 | 0.10 | - | 0.04 | - | - |
| Wheat | 0.54 | 0.65 | 0.48 | 0.60 | 1.4 | 0.54 |
| Rye | 1.93 | 1.89 | 1.48 | 1.65 | 2.4 | 1.53 |
| Triticale | 0.34 | 0.65 | 0.70 | 0.51 | 1.2 | - |
| Sorghum | - | - | 0.26 | - | 1.0 | - |
| Corn | - | - | <0.00 | 0.12 | - | - |

1. Anderson et. al., 1978 J. Inst. Brew. 84, 233-239
2. Henry, R. J. 1985 J. Sci. Food Agri., 36, 1243-1253
3. Joergensen et. al., 1988 AGRIS, Vol. 53(5) p. 287-296
4. McCleary and Glennie-Holmes 1985 J. Inst. Brow. 91, 285-295
5. Prentice et. al., 1980 Cereal Chem. 57: 198-202
6. Young-Tack Lee. 2001 Korean J. Food & Nutri. Vol. 14. No. 3, 233-238

Among the natural materials described above, barley was first selected and used for development of a food product in the present invention, since it comprises lots of dietary fiber which can provide full-stomach feeling and beta-glucan which is useful for prevention and treatment of metabolic syndrome. In terms of physical properties of a product to be developed, advantages and disadvantages of barley are not much different from those of the natural materials described above. Barely is advantageous in that it comprises lots of dietary fiber and high content of beta-glucan, relatively low calories and almost zero fat content. Meanwhile, it is disadvantageous in that it has moderate protein content and high carbohydrate content, and also hard to digest and the taste is not so good.

According to the present invention, said problem can be solved by a fermentation process using microorganisms so that content of carbohydrates, which easily accumulate in a human body, is lowered, content of beta-glucan, which can prevent and treat obesity and metabolic syndrome, is increased and at the same time a digestion property, taste and flavor are dramatically improved.

Brief outlines of a process for producing a barley fermented product according to the present invention are given below.
1. Barley is polished or partially polished, and if necessary, it is used as it is without any polishing.
2. After complete washing, pulverization is carried out using a pulverizer. If necessary, pulverization can be omitted.
3. Pulverized barley is liquefied, saccharified and subjected to a fermentation process. If necessary, a saccharification step can be omitted.
4. Saccharified barley is added to a fermentation tank and added with various materials that are required for growth of microorganisms, and fermentation is carried out. If necessary, only water-soluble components can be fermented after their isolation while water-insoluble components are separately stored and later added back at a certain stage.
5. As for the microorganisms used, yeast is employed, since it has been already proven to be safe and known to be very useful for human.
6. Nucleic acids are controlled either by separating yeast using a centrifuge after the completion of fermentation, or by selecting a fermentation method which can minimize cell mass of the microorganisms. Also, with a direct treatment of a fermented product that is obtained after the completion of fermentation, nucleic acids can be controlled.
7. If only the water-soluble components are fermented after their isolation from the water-insoluble components, the water-insoluble components stored separately are added back to the fermented product in which nucleic acids are controlled. Further, when only the fermented product is to be used, it is not necessary to add the water-insoluble components back to the product.
8. Finally, to the fermented product to which various treatments have been carried out, a treatment to give texture, flavor, taste and the like is applied to obtain a finished food or medicine product. In this case, a step of concentration or drying can be added.

With respect to the processes described above, five representative processes are summarized below.

### Process 1

Raw materials - Pulverization - Liquefaction - Saccharification (can be omitted) - Fermentation - Yeast separation - Concentration or Drying - Adding texture, flavor and taste

### Process 2

Raw materials - Pulverization - Liquefaction - Saccharification (can be omitted) - Fermentation while minimizing production amount of microorganisms - Concentration or Drying - Adding texture, flavor and taste

### Process 3

Raw materials - Pulverization - Liquefaction - Saccharification (can be omitted) - Fermentation of water-soluble components only after their separation from water-insoluble components - Yeast separation - Combining yeast separated solution with the water-insoluble components - Concentration or Drying - Adding texture, flavor and taste

### Process 4

Raw materials - Pulverization - Liquefaction - Saccharification (can be omitted) - Fermentation of water-soluble components only after their separation from water-insoluble components - Yeast separation - Concentration or Drying - Adding texture, flavor and taste

### Process 5

Raw materials - Pulverization - Liquefaction - Saccharification (can be omitted) - Fermentation - Control of nucleic acids by treating a fermented product itself - Concentration or Drying - Adding texture, flavor and taste

A target product will be mostly consumed by people having obesity or a risk of having obesity or people suffering from metabolic syndrome to control their weight and body fats, especially fats accumulated around their intestines. As such, when it is taken in an excess amount, various risks due to increased concentration of uric acid should be taken into consideration, and therefore it is necessary to control nucleic acids in the product of the present invention. In particular, for people who are suffering from gout, nucleic acids high in purine content should be strictly controlled in their everyday diet. Thus, a step of controlling nucleic acids in a developed product is absolutely required.

In addition, since it is suggested by the United Nations to have only 3 to 4g of nucleic acids per day, in order for a normal person to have enough amount of the product of the present invention, a step of controlling nucleic acids is definitely required.

For reference, it has been known that dietary fiber is to be taken in an amount of 24g everyday and proteins are to be taken in an amount of 70g everyday. As such, the maximum level of relative nucleic acid content compared to the final product can be easily calculated.

Control level and a method of controlling nucleic acids may vary depending on composition of a final product. If a process of controlling nucleic acids in which yeast is separated from a fermented product is selected, it is possible that some of the useful substances are also removed along with the yeast due to characteristics of the process.

Meanwhile, although yeast is an excellent food product comprising good-quality proteins, in terms of nucleic acids, it comprises almost seven times the amount of nucleic acids compared to those comprised in a sardine, which is one of the food stuffs having enriched nucleic acids. Specifically, it is known that the nucleic acid content in yeast is almost 6 to 12% in their dry weight.

When a fermentation liquid in which nucleic acids are controlled and water-insoluble components are combined with each other, depending on specification of a final product, their ratio can be changed, and therefor various types of the product can be prepared.

Further, when only water-soluble dietary fiber including beta-glucan is used, water-insoluble components may not be combined with them.

Further, each component can be first concentrated or dried, and then combined with each other, or they can be combined first, and then concentrated or dried together. Still further, any one component can be first concentrated or dried, and then combined with others followed by re-drying.

For giving texture to a concentrated or dried product at the last production stage, various treatment methods can be employed. For example, beef texture, bread texture, snack texture and the like can be provided. A treatment step to give such texture can be achieved by an extrusion molding process. For extrusion molding, composition ratio of raw materials, addition amount of raw materials, moisture content, screw rotation speed, barrel heating temperature, etc. can be adjusted. In this case, extrusion molding can be carried out under the condition in which moisture content is increased to about 40%, and temperature is maintained at about 100 to 170°C and pressure is 100 to 1000 psi. For obtaining protein texture, a cooling die or a circular die can be used.

In order to achieve other purpose of the invention, the present invention provides a fermented product of natural materials produced by the process for producing a fermented product according to the present invention, wherein the fermented product comprises decreased carbohydrate content and increased beta-glucan content. The fermented product of natural materials includes a fermented product in which edible microorganisms are used for fermentation and nucleic acids are controlled. Descriptions about edible microorganisms, natural materials and control of nucleic acids are as given in the above.

The present invention still further provides a product which comprises the fermented product of natural materials of the present invention. The product can be a food product or a medicine product. The food product or medicine product of the present invention may consist of the fermented product of natural materials itself, or may comprise other additives. Examples of additives include protein, fat, carbohydrate, dietary fiber, beta-glucan, inorganic salts, vitamin, etc., but not limited thereto. For a fermentation process of the present invention, an additive can be used. As an inorganic salt, there are MgCl₂, KCl, NaCl, Ca-lactate, iron ammonium citrate, etc. As vitamin, there are vitamin B₁, vitamin B₂, vitamin B₆, nicotinic acid amide, vitamin C, etc. As a nitrogen source, there are sodium nitrate (NaNO₃), ammonia water (NH₄OH), ammonium carbonate ((NH₄)₂CO₃), ammonium chloride (NH₄Cl), ammonium hydrogen phosphate ((NH₄)₂HPO₄), Peptone, Soytone, Tryptone, yeast extract, soy meal, malt extract, etc., but not limited thereto.

A product according to one embodiment of the present invention is **characterized in that** it comprises nucleic acids in an amount of 3 to 4g or less on the basis of 70g of proteins or 24g of dietary fiber in the product. Since it has been suggested to have 70g of proteins and 24g of dietary fiber per day and also the United Nations suggests to have 3 to 4g of nucleic acids per day, it is preferable that in the product of the present invention, nucleic acids are comprised in an amount of 3 to 4g or less on the basis of 70g of proteins or 24g of dietary fiber.

The food product or medicine product according to one embodiment of the present invention can be used for prevention and treatment of obesity and metabolic syndrome. Specifically, because the food product or medicine product according to the present invention is tasty, has low calories and can give full-stomach feeling, they can be used for prevention and treatment of obesity and metabolic syndrome.

The fermented product of the present invention can be obtained by fermentation with any kind of microorganisms, and by performing fermentation with microorganisms, taste and flavor of natural materials can be improved.

Preferably, the microorganisms are yeast and the natural materials can be barley, oat, rye, triticale, sorghum, or a mixture comprising them.

For a product according one embodiment of the present invention, content of beta-glucan on the basis of total weight of the product is preferably 0.26-1.0 weight% or more when the natural materials are sorghum or a mixture comprising sorghum. It is preferably 0.34-1.2 weight% or more when the natural materials are triticale or a mixture comprising triticale. Further, it is 1.48-2.4 weight% or more when the natural materials are rye or a mixture comprising rye. In this regard, a mixture comprising sorghum indicates that it comprises barley, tapioca and the like in addition to sorghum, for example.

For a product according one embodiment of the present invention, content of beta-glucan on the basis of total weight of the product is preferably 2.5-5.7 weight% or more when the natural materials are oat or a mixture comprising oat. Further, it is 4.09-6.3 weight% or more when the natural materials are barley or a mixture comprising barley.

The reason for the limitation of beta-glucan content as described above is that, since the above numbers are the content of beta-glucan before fermentation of each natural materials, the content of beta-glucan comprised in a product obtained after fermentation of each natural materials with microorganisms will increase compared to content of beta-glucan initially comprised in non-fermented natural materials. Therefore, when content of beta-glucan is low, the product of the present invention may further comprise it. In addition, the product of the present invention preferably has protein content that is higher than that comprised in natural materials before fermentation.

Advantages of the present invention compared to conventional commercial techniques are summarized below.
1. By using easily obtainable natural materials or a mixture comprising them, a food product or a medicine product, which is high in beta-glucan content but low in carbohydrate content and can be used for prevention and treatment of obesity and metabolic syndrome, can be produced easily at low cost.
2. By introducing a biochemical change in components which have an adverse effect on taste and flavor and also farting, a property as a food product can be dramatically improved. Considering that one of the reasons for farting is due to gas generated by microorganisms who reside in large intestine and contribute to fermentation of polysaccharides that are undigested and unabsorbed in small intestine, when a product of the present invention which can introduce a biochemical change of some polysaccharides based on fermentation caused by microorganisms is taken, farting can be prevented more compared to a case in which natural materials are consumed as they are.
3. A waste, which is one of the disadvantages confronted in fermentation industry, is not produced.
4. A food product or a medicine product, which is useful for prevention and treatment of obesity and metabolic syndrome and can be consumed as a functional substitute for regular meals, can be easily produced at low cost.

Herein below, the present invention will now be described in greater detail with reference to the following examples. However, it is only to specifically exemplify the present invention and in no case the scope of the present invention is limited by these examples.

### Examples

### Example 1: Production of fermented product of barley

### (1) Raw material (barley) preparation

Commercially available barley was pulverized to average particle size of 500µm by using a pin mill.

### (2) Liquefaction and saccharification

Pulverized barley was dissolved to obtain final solid matter concentration of 13.8% and stirred until the solution becomes homogeneous. After adding α-amylase (Termamyl 120L) in an amount of 1% relative to the barley solid matters while adjusting pH of the solution to 6.3, the mixture was reacted for 3 hours in an incubation bath at 95°C.

The solution obtained after the completion of liquefaction was slowly cooled to 62°C. and pH was adjusted to 4.2 by adding 50% sulfuric acid. Thereafter, for saccharification, amyloglucosidase (AMG 300L) was added in an amount of 2% relative to the barley solid matters, and then the mixture was reacted for 20 hours in an incubation bath at 62°C.

### (3) Separation into water-insoluble components and water-soluble components

The saccharified solution that had been obtained from the above liquefaction and saccharification process was centrifuged at 4,910 x g for 10 minutes (MF 600, Hanil Science Industrial Co., Korea) to separate it into water-insoluble components and water-soluble components, which are 17 weight% and 83 weight%, respectively.

### (4) Culture and fermentation

The microorganism used for this example was baker's yeast (Saccharomyces sp.), which was cultured on YPD solid medium comprising glucose 2% (w/v), peptone 2% (w/v), yeast extract 1% (w/v) and agar 1.5% (w/v) with 4-day culture cycle at 25°C.

Primary seed culture was carried out by inoculating part of the yeast cultured on YPD solid medium to YPD liquid medium comprising glucose 2% (w/v), peptone 2% (w/v), and yeast extract 1% (w/v) (250mℓ, conical flask, 50mℓ working volume) and culturing for 24 hours at 30°C, 150rpm.

Secondary seed culture was carried out by inoculating the seed which had been cultured by the primary seed culture to YPD liquid medium (1% v/v volume) and culturing for 12 hours at 30°C, 150rpm.

For main culture, 5L fermentation tank (Liflus GX, Biotron, KOREA) was used. Sterilization was carried out at 121°C for 20 minutes. To a medium which had been prepared by adding organic nitrogen source (Soytone) to the sample obtained from the above liquefaction and saccharification process (2Kg, 8% saccharified solution + 4% Soytone), 1% (v/v) secondary seed culture was inoculated and cultured for 48 hours under the condition including culture temperature of 30°C, shaking speed of 250rpm and air flow rate of 1 vvm. As a result, a fermentation solution having yeast concentration of 21.5g/L was prepared (based on dry yeast weight). pH was adjusted to 5.5 during the culture by using 28% NH₄OH and 1N HCl.

### (5) Removal of yeast

The yeast culture solution obtained after main culture was centrifuged for 10 minutes at 4,910 x g (MF 600, Hanil Science Industrial Co., Korea) to obtain yeast solution and supernatant, which were 9 weight% and 91 weight%, respectively.

### (6) Concentration

The supernatant from which yeast had been removed was concentrated to the solid matter concentration of 20% by using a reduced-pressure concentrating device (N 3000, Eyela, Japan) under heating condition at 55°C.

### (7) Combining

The water-insoluble components, which had been obtained from a process of separating them from the water-soluble components described above and stored thereafter, and the fermentation concentrate, from which yeast had been removed, were combined with each other.

### (8) Drying

The mixture comprising the solid matter concentration of 23% was dried to the solid matter concentration of 95% under temperature condition including hot air (inlet) at 160°C and outlet air at 95°C, by using a spray dryer (SD-1000, Eyela, Japan).

### (9) Texture

The dried sample obtained from the above was molded using a double-screw extrusion molding machine at barrel heating temperature of 140°C, followed by cooling followed by cooling using a cooling die.

### (10) Paste

To add flavor, taste and special functions to a texturized sample, a small amount of the extracts of squash, potato or sea weed (or kelp) was added and also spices were added separately. After the extrusion molding under high temperature and high pressure condition, the final product was obtained.

Comparison between constitutional components of the initial raw materials and those of the final product is summarized in the following table.

**Table 3**

| | Carbohydrate | Fat | Protein | Dietary fiber (beta-glucan) | Nucleic acids |
|---|---|---|---|---|---|
| Raw barley | 64.0 | 2.1 | 12.0 | 11.2(5.5) | 0.4 |
| Final product | 28.1 | 2.2 | 36.1 | 21.2(8.1) | 0.5 |

As it is shown in the above table, compared to raw barley, the final product of the present invention has dramatically reduced amount of carbohydrates while the content of dietary fiber and the content of beta-glucan are significantly increased. In addition, since the nucleic acid content is much lower than recommended dietary allowance and protein content is more than appropriate level, the product of the present invention can be suitably taken as a substitute for regular meals.

## Claims

1. A method for producing a fermented product of natural materials comprising decreased carbohydrate content and increased beta-glucan content, including:
- adding edible microorganisms to natural materials for fermentation; and
- controlling nucleic acids during or after the fermentation step.

2. The method for producing a fermented product of natural materials comprising decreased carbohydrate content and increased beta-glucan content according to Claim 1, **characterized in that** it includes:
- pulverizing and liquefying natural materials;
- adding edible microorganisms to the pulverized and liquefied natural materials obtained above for fermentation;
- controlling nucleic acids during or after the fermentation step;
- concentrating or drying the resulting fermented product in which nucleic acids are controlled; and
- adding texture, flavor and taste to the resulting concentrated or dried fermented product.

3. The method according to Claim 2, **characterized in that** a saccharification process is further carried out after pulverizing and liquefying the natural materials.

4. The method according to Claim 1, **characterized in that** water-soluble components and water-insoluble components are separated from each other before the fermentation of the natural materials and only the water-soluble components are subjected to the fermentation.

5. The method according to Claim 4, **characterized in that** the separated water-insoluble components are combined with a fermented product that is obtained after the fermentation process.

6. The method according to Claim 1, **characterized in that** the edible microorganisms are yeast.

7. The method according to Claim 1, **characterized in that** the fermentation is aerobic fermentation.

8. The method according to Claim 1, **characterized in that** the fermentation is to minimize a production amount of microorganisms.

9. The method according to Claim 1, **characterized in that** the step for controlling nucleic acid is to remove partially or completely the microorganisms from the product obtained after the completion of fermentation.

10. The method according to Claim 1, **characterized in that**, during the step for controlling nucleic acids, laser irradiation, a heat shock treatment, sonication or alkaline lysis is carried out.

11. The method according to Claim 1, **characterized in that** the natural materials are selected from a group consisting of barley, wheat, triticale, rye, sorghum, oat, rice, corn, adlay, millet, hog millet, potato, and sweet potato.

12. A fermented product of natural materials comprising decreased carbohydrate content and increased beta-glucan content, which is produced by a method of any of Claims 1 to 11.

13. A product which comprises the fermented product of natural materials of Claim 12.

14. The product according to Claim 13, **characterized in that** the product is a food product or a medicine product.

15. The product according to Claim 13, **characterized in that** the product further comprises protein, fat, carbohydrate, dietary fiber, beta-glucan, inorganic salts or vitamins.

16. The product according to Claim 13, **characterized in that** the product comprises nucleic acids in an amount of 3 to 4g or less on the basis of 70g of protein content or 24g of dietary fiber content.

17. The product according to Claim 13, **characterized in that**, on the basis of the total weight of the product, beta-glucan content is 0.26-1.0 weight% or more when the natural materials are sorghum or a mixture comprising sorghum, 0.34-1.2 weight% or more when the natural materials are triticale or a mixture comprising triticale, or 1.48-2.4 weight% or more when the natural materials are rye or a mixture comprising rye.

18. The product according to Claim 13, **characterized in that**, on the basis of the total weight of the product, beta-glucan content is 2.5-5.7 weight% or more when the natural materials are oat or a mixture comprising oat, or 4.09-6.3 weight% or more when the natural materials are barley or a mixture comprising barley.

19. The product according to Claim 17 or 18, **characterized in that** protein content of a product is increased compared to the protein content of natural materials before fermentation.

20. The product according to Claim 17 or 18, **characterized in that** beta-glucan is further comprised.
